# EUROPEAN PATENT APPLICATION

(11) **EP 0 914 817 A1**
(43) Date of publication of application: **12.05.1999**
(21) Application number: 98119883.1
(22) Date of filing: 20.10.1998
(51) Int. Cl.: A61K 7/48

(54) **Nipple ointment composition**

(30) Priority: 20.10.1997 EP 97118149
(71) Applicant: Chemisch Adviesbureau Drs. J.C.P. Schreuder B.V., NL-3740 AK Baarn (NL)
(72) Inventor: Schreuder, Johannes Carolus Petrus, 1406 NC Bussum (NL)
(74) Representative: DIEHL GLAESER HILTL & PARTNER

(57) **Abstract**

There is provided a nipple ointment composition comprising:
(a) a natural edible plant fat, containing glycerides of natural saturated and/or unsaturated fatty acids, in an amount, relative to the weight of the total compositions, of from 65 to 80 wt%,
(b) a branched hydrocarbon oil in an amount, relative to the weight of the total composition, of from 10 to 30 wt%,
(c) an oil, containing mono ethylenic acyl alcohol esters derived from natural saturated and unsaturated fatty acids, in an amount, relative to the weight of the total composition, of from 1 to 10 wt%,
(d) allantoin in an amount, relative to the weight of the complete composition, of from 0.5 to 2 wt%, and
(e) a naturally occurring preservative in an amount, relative to the weight of the complete composition, of from 0.5 to 3 wt%,
   wherein the respective percentages of the components (a) through (e) will add up to 100 wt%.

## Description

The invention relates to a nipple ointment composition and to the application of said composition.

In particular the invention relates to a multifunctional nipple ointment composition for the treatment of chapped, sore and/or even inflamed sensitive skin parts of the nipples themselves and of the surrounding area, i.e. the areola.

It is known that during the last month of the pregnancy, the nipples can become moist, which without effective treatment leads to irritation, and finally to dryness.

Moreover, said affections of the nipples and the areola can frequently occur in the post natal period and in particular when breast feeding is provided.

Many compositions have already been proposed for this purpose in the course of the last decade.

However, the up till now available prior art compositions had as disadvantage that they had been composed of constituents, which significantly disturb the biological equilibrium of the affected skin parts.

As a consequence, said prior art compositions usually did not relieve the irritation or even pain and certainly did not stop these affections or even heal the affected skin parts in an efficient way.

Therefore an object of the present invention is to provide a multifunctional nipple ointment composition which combines an effective prevention of the hereinbefore mentioned affections and of the disturbance of the biological equilibrium and which does not cause a burning, irritated and/or inflamed feeling.

As result of extensive research and experimentation a nipple ointment composition aimed at achieving the above has surprisingly been found.

Accordingly the present invention provides an effective nipple ointment composition which comprises at least:
(a) a natural edible plant fat, comprising di- and/or triglycerides of natural saturated and/or unsaturated fatty acids, containing of from 14 to 24 carbon atoms, in an amount, relative to the weight of the total composition, of from 65 to 80 wt% and preferably from 70 to 75 wt%,
(b) a stable, inert, homogeneous liquid branched hydrocarbon oil, having from 20 to 40 carbon atoms and preferably from 26 to 32 carbon atoms, in an amount, relative to the weight of the total composition, of from 10 to 30 wt% and preferably from 15 to 25 wt%,
(c) an oil, comprising mono ethylenic acyl alcohol esters derived from natural saturated and unsaturated fatty acids, containing from 14 to 24 carbon atoms and preferably mainly unsaturated fatty acids, in an amount, relative to the weight of the total composition, of from 1 to 10 wt% and preferably from 4 to 7 wt%,
(d) allantoin (5-ureidohydantoin or glyoxyldiureide) in an amount of, relative to the weight of the complete composition, from 0.5 to 2 wt% and preferably from 0.8 to 1.5 wt%,
(e) a naturally occurring preservative in an amount of, relative to the weight of the complete composition, from 0.5 to 3 wt% and preferably from 0.8 to 2 wt%.

It will be appreciated that the respective percentages of components (a) through (e) will add up to 100 wt%.

In addition to the hereinbefore specified primary ingredients (a) up to (e), one or more secondary ingredients can be included in the compositions of the present invention.

Such secondary ingredients are not strictly necessary for the efficacy of the compositions of the present invention, but can be included in only small amounts in order to reach optimal properties.

Examples of said secondary ingredients are perfumes, flavouring agents and the like, which may be selected from a great variety of commercial compounds and preferably from those which are occurring in nature.

Preferably as secondary ingredient ethyl vanillin is used in an amount, relative to the weight of the complete composition, of from 0.01 to 0.1 wt% and more preferably from 0.03 to 0.06 wt%.

As preferred ingredient (a) shea butter is included and more preferably a shea butter which has been obtained via an extraction with hexane as solvent.

However it will be appreciated that also shea butter, obtained via a mechanical crushing process, i.e. shea butter which is substantially free of extracting solvent, can be used.

The main function of said ingredient (a) is that of an emollient.

A preferred shea butter ingredient (a) is Cetiol SB 45 (trademark).

As preferred ingredient (b) squalane (also known as spinacene, dodecahydrosqualene, perhydrosqualene, and 2,6,10,15,19,23-hexamethyltetracosane) is included as colourless, odourless, tasteless, transparent, stable homogeneous liquid oil (at ambient temperature).

It has been found that squalane represents a natural emollient, which imparts to the skin a suppleness without an unpleasant greasy feeling.

Moreover it has been found that squalane, when applied to washed or sun-exposed skin, helps to restore the naturally occurring lost oils.

More preferably commercial grades of squalane are used, which have been obtained by direct hydrogenation of shark liver oils which may contain some butyl alcohol.

As preferred ingredient (c) Jojoba oil (or Buxus chinensis) is included, composed of esters derived from erucic acid, oleic acid, gadoleic acid as major components (up to 93 wt%) and palmitic acid, palmitoleic acid, linoleic acid and behenic acid as minor components (up to 6 wt%).

More preferably an Jojoba oil, wherein more than 95 wt% of the esters occur as wax esters, is used.

As preferred ingredient (e) Gamma-Oryzanol (e1) is included which serves as antioxidant/preservative and which is consisting of a mixture of phyto sterols (beta/sito sterol, campestrol), cyclo artenol, stigmasterol and ferulic acid.

It will be appreciated that also mono-, di- and/or triglycerides of capric acid, caproic acid and/or caprylic acid can be included in the present nipple ointment compositions as antibioticum/preservative (e2).

Preferably mono-, di-, and/or triglycerides of capric acid and/or caprylic acid comprising a predominant proportion of monoglycerides and diglycerides and minor amounts of triglycerides are used, in addition to the beforementioned antioxidant/preservative such as Gamma-Oryzanol.

An example of said preservative is the commercial glyceryl caprylate/glyceryl caprate mixtures of the tradename "Imwitor 988".

A preferred embodiment of the present nipple ointment composition comprises:
(a) shea butter in an amount, relative to the weight of the total composition, of from 70 to 75 wt%,
(b) squalane, in an amount relative to the weight of the total composition, of from 18 to 22 wt%,
(c) Jojoba oil (Buxus chinensis), in an amount relative to the weight of the total composition, of from 4 to 6 wt%,
(d) allantoin, in an amount relative to the weight of the total composition, of from 0.8 to 1.5 wt%,
(e1) Gamma-Oryzanol, in an amount relative to the weight of the total composition, of from 0.8 to 1.5 wt%,
(e2) glyceryl caprylate/caprate mixtures, comprising major proportions of mono- and diglycerides, in an amount relative to the weight of the total composition, of from 1.5 to 2.5 wt%,
(f) ethyl vanillin, in an amount relative to the weight of the total composition, of from 0.03 to 0.06 wt%,
   wherein the respective percentages of the components (a) through (f) will add up to 100 wt%.

The nipple ointment compositions can be prepared by methods known in the art, i.e. by mixing the ingredients together and homogenising the mixture, if necessary under gently heating one or more of the ingredients.

Preferably the present nipple ointment compositions are prepared by heating the components (a), (b), (c), and (e2) to a temperature of up to 60°C, and adding slowly the ingredient Gamma-Oryzanol (e1) until complete dissolution, addition of the allantoin (d), followed by homogenising the mixture during 20 to 30 minutes.

Thereafter the obtained solution is cooled to 40°C, whereafter the ethyl vanillin (f) is added, under stirring until complete dissolution.

Thereafter the mixture is cooled to ambient temperature and further homogenised.

It will be appreciated that another aspect of the present invention is formed by the application of the hereinbefore specified nipple ointment compositions.

During the pre-natal period the nipples and areola can be treated twice a day, after cleaning with water and optionally soap and patting dry. An amount of from 0.5 to 2 grams of the composition of the present invention can be applied, while during the post-natal period the same amount of said composition can be applied to the nipples and areola after each feeding and after cleaning with water and optionally soap and patting dry.

The invention is illustrated by the following examples, however without restricting its scope to these specific embodiments.

### Example 1

Under stirring at a temperature of 60°C , the following ingredients were combined into a nipple ointment composition:

| | |
|---|---|
| Shea butter | 73 wt% |
| Squalane | 20 wt% |
| Jojoba oil | 4.95 wt% |
| Gamma-Oryzanol | 1 wt% |
| Allantoin | 1 wt% |
| Ethyl vanillin | 0.05 wt% |

By application twice a day of amounts of 0.5 g each on the nipple and areola, dryness and irritation could be prevented in the prenatal period.

### Example 2

Under stirring at a temperature of 50°C the following ingredients were combined into a nipple ointment composition:

| | |
|---|---|
| Shea butter | 70 wt% |
| Squalane | 22 wt% |
| Jojoba oil | 6 wt% |
| Gamma-Oryzanol | 1.4 wt% |
| Allantoin | 0.55 wt% |
| Ethyl vanillin | 0.05 wt% |

Application of amounts of 0.7 g each after every breast feeding on a precleaned and dried nipple and areola, prevented successfully dryness and irritation.

### Example 3

Under stirring at a temperature of 50°C the following ingredients were combined into an efficient nipple ointment composition:

| | |
|---|---|
| Shea butter | 77 wt% |
| Squalane | 17 wt% |
| Jojoba oil | 4 wt% |
| Allantoin | 1.2 wt% |
| Gamma-Oryzanol | 0.75 wt% |
| Ethyl vanillin | 0.05 wt% |

### Example 4

Under stirring the following ingredients were combined:

| | |
|---|---|
| Shea butter | 71 wt% |
| Squalane | 20 wt% |
| Jojoba oil | 5 wt% |
| Imwitor 988 | 2 wt% |

After heating to a temperature of about 60°C the Gamma-Oryzanol (1 wt%) was slowly added under continued stirring until complete dissolution.

Thereafter allantoin (0.95 wt%) was added.

The obtained mixture was homogenised during 20 minutes . The mixture was cooled to 40°C, whereafter ethyl vanillin (0.05 wt%) was added and dissolved. The mixture was cooled to ambient temperature and homogenised during 30 minutes.

## Claims

1. Nipple ointment composition, which comprises at least:
(a) a natural edible plant fat, comprising di- and/or triglycerides of natural saturated and/or unsaturated fatty acids, containing of from 14 to 24 carbon atoms, in an amount, relative to the weight of the total compositions, of from 65 to 80 wt%, and preferably from 70 to 75 wt%,
(b) a stable, inert, homogeneous, liquid, branched hydrocarbon oil, having from 20 to 40 carbon atoms and preferably from 26 to 32 carbon atoms, in an amount, relative to the weight of the total composition, of from 10 to 30 wt% and preferably from 15 to 25 wt%,
(c) an oil, comprising mono ethylenic acyl alcohol esters derived from natural saturated and unsaturated fatty acids, containing from 14 to 24 carbon atoms and preferably mainly unsaturated fatty acids, in an amount, relative to the weight of the total composition, of from 1 to 10 wt% and preferably from 4 to 7 wt%,
(d) allantoin (5-ureidohydantoin or glyoxyldiureide) in an amount, relative to the weight of the complete composition, of from 0.5 to 2 wt% and preferably from 0.8 to 1.5 wt%, and
(e) a naturally occurring preservative in an amount, relative to the weight of the complete composition, of from 0.5 to 3 wt% and preferably from 0.8 to 2 wt%,
wherein the respective percentages of the components (a) through (e) will add up to 100 wt%.

2. Nipple ointment composition according to claim 1, characterised in that as ingredient (a) shea butter is included.

3. Nipple ointment composition according to claim 1 or 2, characterised in that as ingredient (b) squalane is included.

4. Nipple ointment composition according to any of claims 1 to 3, characterised in that as ingredient (c) Jojoba oil is included.

5. Nipple ointment composition according to any of claims 1 to 4, characterised in that as ingredient (e) Gamma-Oryzanol is included.

6. Nipple ointment composition according to any of claims 1 to 5, characterised in that it comprises:
(a) shea butter in an amount, relative to the weight of the total composition, of from 70 to 75 wt%,
(b) squalane, in an amount relative to the weight of the total composition, of from 18 to 22 wt%,
(c) Jojoba oil (Buxus chinensis), in an amount relative to the weight of the total composition, of from 4 to 6 wt%,
(d) allantoin, in an amount relative to the weight of the total composition, of from 0.8 to 1.5 wt%,
(e1) Gamma-Oryzanol, in an amount relative to the weight of the total weight composition, of 0.8 to 1.5 wt%,
(e2) glyceryl caprylate/caprate mixtures, comprising major proportions of mono- and diglycerides, in an amount relative to the weight of the total composition of from 1.5 to 2.5 wt%,
(f) ethyl vanillin, in an amount relative to the weight of the total composition of from 0.03 to 0.06 wt%.

7. Use of the compositions according to claims 1 to 6 as a nipple ointment composition.

8. Use of the compositions according to claim 7, characterised in that nipples and areola are treated in the prenatal period twice a day after cleaning with water and patting dry, with an amount of the nipple ointment of from 0.5 to 2 grams.

9. Use of compositions according to claim 7, characterised in that during the post natal period an amount of from 0.5 to 2 grams is applied after cleaning of the nipples and areola after each breast feeding.
